# EUROPÄISCHE PATENTSCHRIFT

(11) **EP 1 100 561 B1**
(45) Veröffentlichungstag und Bekanntmachung des Hinweises auf die Patenterteilung: **13.10.2004**
(21) Anmeldenummer: 99938336.7
(22) Anmeldetag: 26.07.1999
(51) Int. Cl.: A61M 1/34, A61F 2/02, C12M 3/06

(54) **HARNSTOFFERZEUGENDER ORGANERSATZ**
UREAPOIETIC ORGAN REPLACEMENT
SUCCEDANE ORGANIQUE PRODUCTEUR D'UREE

(30) Priorität: 26.07.1998 DE 19833562
(43) Veröffentlichungstag der Anmeldung: 23.05.2001
(73) Patentinhaber: Rennebeck, Klaus, 73240 Wendlingen (DE); Scheller, Albert, 70806 Kornwestheim (DE)
(72) Erfinder: Rennebeck, Klaus, 73240 Wendlingen (DE); Scheller, Albert, 70806 Kornwestheim (DE)
(74) Vertreter: Patentanwälte Hosenthien-Held und Dr. Held
(86) Internationale Anmeldenummer: PCT/EP1999/005317
(87) Internationale Veröffentlichungsnummer: WO 2000/006218

(56) Entgegenhaltungen:
- WO-A-89/11529
- US-A- 5 516 691

## Beschreibung

Die Erfindung betrifft einen bionischen Organersatz, d.h. eine "künstliche Leber", sowohl für den inkorporalen als auch für den extrakorporalen Einsatz.

Wie dies allgemein bekannt ist, finden in der menschlichen Leber verschiedene Stoffwechselvorgänge statt. So werden in den Hepatozyten der Leber insbesondere die Verdauungsprodukte gespeichert und verarbeitet. Darüber hinaus kommt der Leber als Entgiftungsorgan eine wesentliche Bedeutung zu.

Die verschiedenen Strukturen der Hepatozyten, das Cytoplasma, das feine und rauhe endoplasmatische Reticulum, Lysosomen, Peroxisomen und der Golgi-Apparat mit den Mitochondrien, nehmen jeweils unterschiedliche Funktionen wahr. Als Beispiel hierfür sei die Erzeugung von Harnstoff, dem Diamid der Kohlensäure, in der mitochondrialen Matrix der Hepatozyten genannt. Auch die Bildung von Galle, d.h. Gallensalzen und -pigmenten, gehört zu den Aufgaben der Leber. Die als Sekret von den Hepatozyten abgesonderte Gallenflüssigkeit wird von den interlobulären Gallenkapillaren aufgenommen und den interhepatischen Gallengängen zugeführt.

In der US 5,516,691 ist ein Modul zum Kultivieren von Mikroorganismen beschrieben, das aus einem äußeren Gehäuse und drei unabhängigen Membransystemen besteht, von denen mindestens zwei als Hohlfasermembranen ausgebildet sind. Die Hohlfasermembranen bilden ein Netzwerk, in desen Hohlräumen sich Mikroorganismen befinden. Dieses bekannte Modul ist jedoch aufgrund seines Aufbaus nur für den extrakorporalen Einsatz geeignet.

Es ist die Aufgabe der vorliegenden Erfindung, einen Organersatz anzugeben, der bei Vorliegen von Organinsuffizienz oder -versagen die Funkionen derselben zumindest teilweise übernehmen kann und der sowohl für den intrakorporalen als auch für den extrakorporalen Einsatz geeignet ist.

Diese Aufgabe wird erfindungsgemäß durch einen harnstofferzeugenden Organersatz gemäß Anspruch 1 gelöst.

Der erfindungsgemäße bionische Organersatz weist eine Struktur drei Gruppen textiler Mikrohohlfasern auf, wobei die Mikrohohlfasern jeder Gruppe in mindestens einen jeweiligen zentralen Flüssigkeitsleiter münden, wobei die Mikrohohlfasern der ersten Gruppe aus protonenleitendem Material hergestellt sind und Perforationen für die Ableitung von Gallenflüssigkeit in das Innere der Fasern aufweisen und die eine der Oberflächen im wesentlichen jeder Mikrohohlfaser der ersten Gruppe hydrophil und lipophil ausgebildet ist, während die andere der Oberflächen hydrophob und lipophob ausgebildet ist, und wobei auf der äußeren Oberfläche aller Mikrohohlfasern Zellkulturen anzüchtbar sind.

Der erfindungsgemäße Organersatz ist so gestaltet, daß er, wenn auf den Oberflächen der Mikrohohlfasern Hepatozyten angezüchtet sind, im wesentlichen alle Aufgaben der Leber sowohl intra- als auch extrakorporal erfüllen kann. Die Mikrohohlfasern der Struktur weisen textile Eigenschaften auf, d.h., sie sind sehr fein und flexibel. Sie bilden die Kapillargefäße der künstlichen Leber.

Die menschliche Leber ist aus ca. 1 bis 1,5 Millionen Leberläppchen (Lobuli hepati) aufgebaut, die eine Höhe von etwa 2 mm und einen Durchmesser von etwa 1 bis 3 mm aufweisen und aus den Leberzellen (Hepatozyten) bestehen, welche näherungsweise polyedrisch angeordnet sind. Die in den Leberläppchen gebildete Galle wird als Sekret über die interlobulären Gallenkapillaren und die kleinen Gallengänge in die Gallenblase (Vesica fellea) bzw. über den Gallengang (Ductus chloledochus) direkt in den Dünndarm drainiert. Die interlobulären Gallenkapillaren weisen in der Regel einen Durchmesser von etwa 0,1 bis 1,5 µm auf, können sich jedoch bei Behinderung des Gallenflusses auf ca. 15 bis 20 µm ausdehnen.

Die Funktionen der interlobulären Gallenkapillaren werden bei dem erfindungsgemäßen Organersatz von den Mikrohohlfasern der ersten Gruppe wahrgenommen. Ebenso wie die natürlichen Gallenkapillaren müssen sie aus einem Material hergestellt sein, das protonenleitend ist. Als geeignetes Material hierfür haben sich beispielsweise Apatit sowie einige Polymere, insbesondere Polytetrafluorethylen, erwiesen. Um eine möglichst große Oberfläche für die Zellanzucht zur Verfügung zu stellen, werden spongiöse Mikrohohlfasern bevorzugt. Darüber hinaus müssen die Fasern so strukturiert sein, daß sie selektiv für die beteiligten Metaboliten durchlässig, d.h. semipermeabel, sind.

Eine weitere Anforderung besteht darin, daß eine der Oberflächen jeder Mikrohohlfaser der ersten Gruppe, vorzugsweise die innere Oberfläche, lipophob und hydrophob und die andere, vorzugsweise die äußere, Oberfläche lipophil und hydrophil ausgebildet ist. Auf diese Weise läßt sich vermeiden, daß die in den Hepatozyten gebildete Galle die Zellen zersetzt. Die Oberflächen der Mikrohohlfasern lassen sich beispielsweise durch eine Beschichtung mit geeigneten Polymeren oder Keramiken lipophob bzw. hydrophob machen, d.h. "versiegeln". Die diesbezügliche Technik ist dem Fachmann auf diesem Gebiet bekannt und wird deshalb hier nicht näher erläutert.

Für die Drainage der Galle in die Mikrohohlfasern der ersten Gruppe sind diese mit Perforationen über die gesamte Wandstärke versehen. Die Perforationen werden bei der Herstellung in die Mikrohohlfasern durch Recken und Lasern eingebracht. Durch die Perforationen tritt das Gallensekret in die Mikrohohlfasern der ersten Gruppe, welche die synthetischen Gallenkapillaren darstellen, ein und wird von dort einem oder mehreren Flüssigkeitsleitern, die synthetische kleine Gallengänge darstellen, zugeführt.

Neben den Kapillaren und Gängen für die Galle weist die menschliche Leber von der Leberschlagader (Arteria hepatica) und der Pfortader (Vena portae) kommende Gefäßäste auf, die die Leberläppchen mit Blut versorgen. Die Oberflächen der Hepatozyten sind partiell mit Zell- bzw. Plasmamembranen umschlossen, die als Trennwand zu den benachbarten Hepatozyten fungieren, wobei der Abstand etwa 100 bis 200 Å beträgt. Zwischen den sternenförmig auf die Zentralvene (Vena centralis) zulaufenden Hepatozyten sind sogenannten Lebersinusoide mit einem gleichwertigen Durchmesser von etwa 9 bis 12 µm ausgebildet, in denen das von den Gefäßästen kommende Blut zur Zentralvene fließt. Zwischen der für Blutplasma und Makromoleküle permeablen Sinusoidwand und den Hepatozyten befinden sich die sogenannten Disse-Räume.

Erfindungsgemäß werden die von der Pfortader und der Leberarterie kommenden Äste bzw. Kapillaren der menschlichen Leber von den Mikrohohlfasern der zweiten und dritten Gruppe gebildet. Diese weisen entsprechen den Abmessungen der realen Äste einen Innendurchmesser von etwa 1 bis 150 µm auf. Gemäß den Eigenschaften der Äste der Pfortader und der Leberarterie weisen die Mikrohohlfasern der zweiten und dritten Gruppe eine geringere Protonenleitfähigkeit auf und wirken als Ionen- oder Elektrolyttrennmembranen. Als geeignete Materialien hierfür haben sich insbesondere Keramik und Polymere erwiesen. Die Mikrohohlfasern der zweiten und dritten Gruppe münden jeweils in einen zentralen Flüssigkeitsleiter, der synthetischen Pfortader bzw. der synthetischen Leberarterie. Diese können aus für künstliche Gefäße üblichen biokompatiblen Materialien hergestellt sein.

Auf dem erfindungsgemäßen Organersatz werden, vorzugsweise in vitro, menschliche Leberzellen angezüchtet, bis die äußeren Oberflächen der Mikrohohlfasern vollumfänglich mit humanen Leberzellen bewachsen sind. Die Leberzellen oder Hepatozyten weisen in ihrem Zentrum den Nukleolus auf Darüber hinaus weisen die Leberzellen die sogenannten Mitochondrien auf, die mit multi-semipermeablen Membranen mit einer Trennschärfe (Cut off) im Mikro-, Ultra- bzw. Nanobereich ausgestattet sind mit Größenausschluß und Ladungsausschluß oder einer Kombination von beiden und in denen u.a. enzymatische Reaktionen zur Energielieferung stattfinden. Schließlich befinden sich in den Leberzellen in Sequenzen die Golgi-Apparate, in deren sogenannten Zysternen Lipide vorkommen können.

Sobald ein ausreichender Zellbewuchs vorliegt, kann der erfindungsgemäße Organersatz operativ unter Einsatz von Immunotherapeutika in den Körper eingebracht werden. Dabei ist es möglich, die Medikamente in das Innere der Mikrohohlfasem eingeleitet werden. Selbstverständlich kann die erfindungsgemäße künstliche Leber auch als extrakorporaler Organersatz verwendet werden.

Eine prinzpiell mögliche Alternative zur In-Vitro-Zellanzüchtung stellt die Einbringung des erfindungsgemäßen Organersatzes, in den menschlichen Körper dar, so daß noch gesunde Leberzellen die Mikrohohlfaserstruktur inkorporal bewachsen. Diese Vorgehensweise empfielt sich insbesondere bei nur partieller Leberschädigung und kann gegebenenfalls endoskopisch erfolgen.

Die Mikrohohlfasern des erfindungsgemäßen Organersatzes weisen einen Innendurchmesser von etwa 0,1 bis 50 µm auf. Dies entspricht dem Innendurchmesser der natürlichen Gefäße. Prinzipiell können bei Bedarf auch künstliche Gefäße mit anderen Abmessungen eingesetzt werden, wenn dies sinnvoll und technisch machbar ist. Mikrohohlfasern dieses Durchmessers können z.B. durch Spinnverfahren hergestellt werden. Bezüglich der Details der Eigenschaften, der möglichen Ausgangsmaterialien sowie der Herstellungsverfahren sei auf die internationale Anmeldung WO97/26225 verwiesen. Wie dieser Druckschrift zu entnehmen ist, lassen sich Mikrohohlfasern der angegebenen Dimension mit hoher Präzision herstellen. So ist es möglich, die Schwankungen von Durchmesser und Wandstärke der Mikrohlfasern in einem Bereich von ±6% zu halten, wodurch eine einheitliche Struktur gewährleistet werden kann.

Vorzugsweise sind die Mikrohohlfasern zu einer im Querschnitt bienenwabenförmigen Struktur angeordnet. Dies entspricht wiederum dem natürlichen Aufbau des Gewebsgerüstes um die einzelnen Leberläppchen. Allgemein kann gesagt werden, daß die Struktur der durch die Mikrohohlfasern und die zugehörigen Flüssigkeitsleiter möglichst weitgehend der natürlichen Struktur der entsprechenden Gefäße angepaßt werden sollte, um beim inkorporalen Einsatz des erfindungsgemäßen Organersatzes die größtmögliche Kompatibilität zu erhalten. Alternativ zur Bienenwabenstruktur ist auch eine Wellpappenförmige Anordnung denkbar, bzw. jede andere Struktur, welche zu einer möglichst großen Oberfläche des Zellbewuchses führt. Die Größe einer Bienenwabenzelle bzw. einer Zelle der Wellpappenstruktur entspricht vorzugsweise etwa derjenigen eines menschlichen Leberläppchens.

Die Gallenflüsse der Leber bestehen aus etwa 97% Wasser, 0,7% Gallensalz, 0,2% Gallenpigmente, 0,06% Cholesterin, 0,7% anorganischen Salzen, 1% Fettsäuren, Lecitin und 0,1% Fett. Der Leberstoffwechsel wird von der Aktivität des Menschen, von dem Grundenergiebedarf sowie von der Nahrungsmittelzufuhr bestimmt. Im Durchschnitt werden etwa 600ml Gallenflüssigkeit pro Tag erzeugt. Aus diesem Grund ist die Zahl und Größe der Perforationen vorzugsweise so ausgelegt ist, daß ein Gallefluß von etwa 100 bis 3000 ml/d ausgelegt. Natürlich kann die Auslegung auch für eine geringere Menge an Gallenflüssigkeit erfolgen, z.B. wenn durch den erfindungsgemäßen Organersatz lediglich ein Teil der menschlichen Leber substituiert werden soll. Im Regelfall hat sich ein Durchmesser der Perforationen, der etwa gleich dem Durchmesser des Filamentlumens der Mikrohohlfaser ist, als ausreichend erwiesen, wobei die Gesamtfläche der Perforationen etwa 30% der Faseroberfläche ausmachen sollten.

Vorzugsweise sind die Mikrohohlfasern der Struktur spätdegradationsfähig oder einstellbar degradierend. Dadurch kann die Lebensdauer der Mikrohohlfasem auf das Lebensalter derjenigen Person, deren Leber durch den erfindungsgemäßen Organersatz substituiert werden soll, eingestellt werden.

Gemäß einer besonders bevorzugten Ausführungsform umfaßt der erfindungsgemäße Organersatz einen portokavalen Shunt. Dies hat den Vorteil der Redundanz.

In ähnlicher Weise können auch die Flüssigkeitsleiter, die der Pfortader bzw. der Lebervene entsprechen mit einem Absperrorgan ausgebildet sein. Aufbau, Herstellung und Anbringung solcher Absperrorgane bzw. Shunts in künstlichen Gefäßen sind im Stand der Technik bekannt, weswegen auf die diesbezüglichen Details nicht näher eingegangen wird.

Der erfindungsgemäße Organersatz kann in beliebiger Größe hergestellt werden, d.h., daß sowohl eine vollständige Leber als auch nur ein Teil derselben durch den Organersatz substituiert werden kann. Die erfindungsgemäße künstliche Leber wird vorzugsweise als integraler Festbett-reaktor mit einer Blutverweildauer von etwa 5 bis 250 Minuten betrieben, wobei durch die Eigenschaften der Mikrohohlfasern, insbesondere aufgrund deren textiler Eigenschaften das Auftreten von Hämostase weitgehend ausgeschlossen werden kann. Bei extrakorporalem Einsatz des erfindungsgemäßen Organersatzes kann eine der Dialyse entsprechende Behandlungsweise gewählt werden.

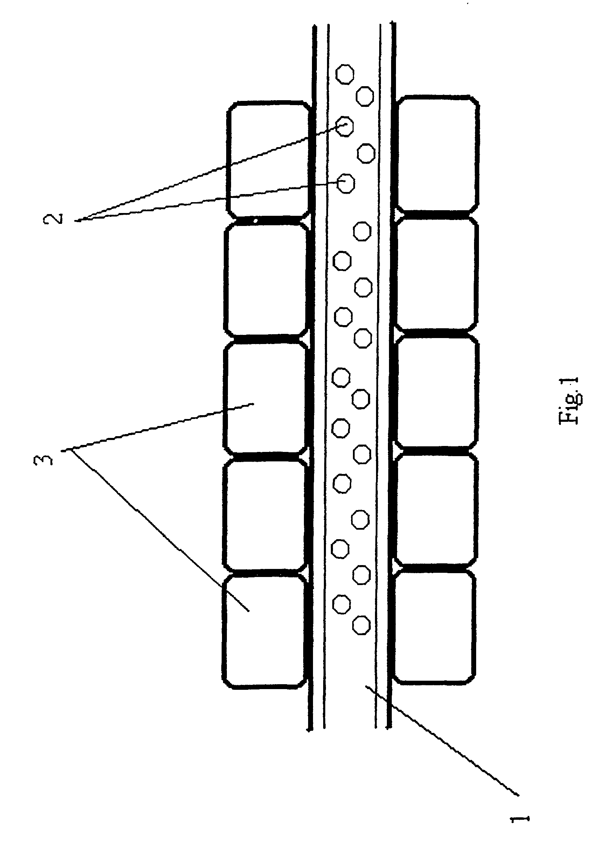

Die Erfindung wird nachstehend mit Bezug auf die beigefügte Zeichnung beschrieben, die als nicht beschränkendes Ausführungsbeispiel gegeben ist und deren einzige Figur einen Querschnitt durch eine Mikrohlfaser 1 der ersten Gruppe, welche eine synthetische interlobuläre Gallenkapillare darstellt. Die Mikrohohlfaser 1 ist aus spongiösem Material, das einen gutes Zellwachstum ermöglicht. In den Wänden der Mikrohohlfaser 1 sind Perforationen bzw. Lochungen 2 eingebracht, die der Ableitung von Gallenflüssigkeit in das Lumen der Mikrohohlfaser 1 dienen. Der Gallenfluß wird durch die lipophile und hydrophile Beschaffenheit der äußeren Oberfläche der Mikrohohlfasern sowie durch die lipophobe und hydrophobe Beschaffenheit der inneren Oberfläche derselben begünstigt.

Auf der Faser sind schematisch die Leberläppchen 3 gezeigt, welche die Leberzellen umfassen. Die durch die Perforationen 2 in die Mikrohlfasern 1 eingedrungene Gallenflüssigkeit wird weiter zu den kleinen Gallengängen (in der Figur nicht dargestellt) transportier, von wo sie, wenn sich der erfindungsgemäße Organersatz im menschlichen Körper befindet, in die Gallenblase oder über den Gallengang direkt in den Dünndarm gelangt.

Durch das beschriebene Gallendrainagesystem wird eine künstliche Leber geschaffen, die sowohl extrakorporal als auch inkorporal eingesetzt werden kann, da die Gallenflüssigkeit nicht die Leberzellen zersetzt. Damit wird ein weitestgehend der natürlichen Struktur und Funktion der menschlichen Leber angepaßter Organersatz bereitgestellt.

Die Mikrohohlfasem 1 der ersten Gruppe, die die synthetischen interlobularen Gallenkapillaren darstellen sind mit den Mikrohohlfasem der zweiten und dritten Gruppe so vernetzt, daß ein dem natürlichen Kapillarnetz um jedes Leberläppchen nachempfundene Struktur entsteht. Die Mikrohohlfasern können zu diesem Zweck entweder als Gewebe oder als Spinnvlies bzw. Spannbond ausgebildet werden.

Es ist darauf hinzuweisen, daß, wenn vorstehend der Ausdruck "menschliche Leber" verwendet wurde, dies keinesfalls beschränkend auszulegen ist und daß der Einsatz des erfindungsgemäßen Organersatzes keinesfalls auf den Menschen beschränkt ist sondern auch Tiere mit ähnlichem Bau der Leber ausgedehnt werden kann. Selbstverständlich müssen in diesem Fall möglicherweise Modifikationen bezüglich Größe und/oder Struktur des erfindungsgemäßen vorgenommen werden. Allgemein kann hierfür der Grundsatz aufgestellt werden, daß der Organersatz möglichst genau den tatsächlichen Verhältnissen des zu substituierenden Organs angepaßt werden sollte.

## Patentansprüche

1. Bionischer Organersatz, aufweisend eine Struktur aus drei Gruppen textiler Mikrohohlfasern, wobei die Mikrohohlfasern jeder Gruppe in einen jeweiligen zentralen Flüssigkeitsleiter münden, wobei auf der äußeren Oberfläche aller Mikrohohlfasern Zellkulturen anzüchtbar sind, **dadurch gekennzeichnet, dass**
die Mikrohohlfasern (1) der ersten Gruppe aus protonenleitendem Material hergestellt sind und Perforationen (2) für die Ableitung von Gallenflüssigkeit in das Innere der Fasern (1) aufweisen und die eine der Oberflächen im wesentlichen jeder Mikrohohlfaser (1) der ersten Gruppe hydrophil und lipophil ausgebildet ist, während die andere der Oberflächen hydrophob und lipophob ausgebildet ist.

2. Bionischer Organersatz nach Anspruch 1, dadurch gekennnzeichnet, daß die äußeren Oberflächen der Mikrohohlfasern vollumfänglich mit humanen, in vitro angezüchteten Leberzellen bewachsen sind.

3. Bionischer Organersatz nach Anspruch 1 oder 2, **dadurch gekennzeichnet, daß** die Mikrohohlfasern einen Innendurchmesser von etwa 0,1 bis 50 µm aufweisen.

4. Bionischer Organersatz nach einem der vorstehenden Ansprüche, **dadurch gekennzeichnet, daß** die Mikrohohlfasern zu einer im Querschnitt bienenwabenförmigen Struktur angeordnet sind.

5. Bionischer Organersatz nach einem der vorstehenden Ansprüche, **dadurch gekennzeichnet, daß** die Flüssigkeitsleiter zu der Struktur der entsprechenden menschlichen Gefäße kompatibel sind.

6. Bionischer Organersatz nach einem der vorstehenden Ansprüche, **dadurch gekennzeichnet, daß** die Zahl und Größe der Perforationen (2) so ausgelegt ist, daß ein Gallefluß von etwa 100 bis 3000 ml/d möglich ist

7. Bionischer Organersatz nach einem der vorstehenden Ansprüche, **dadurch gekennzeichnet, daß** die Mikrohohlfasern der Struktur spätdegradationsfähig oder einstellbar degradierend sind.

8. Bionischer Organersatz nach einem der vorstehenden Ansprüche, **dadurch gekennzeichnet, daß** er einen portokavalen Shunt umfaßt.

9. Bionischer Organersatz nach einem der vorstehenden Ansprüche, **dadurch gekennzeichnet, daß** der Flüssigkeitsleiter, der der Lebervene entspricht, mit einem Absperrorgan ausgebildet ist.

10. Bionischer Organersatz nach einem der vorstehenden Ansprüche, **dadurch gekennzeichnet, daß** der Flüssigkeitsleiter, der der Pfortader entspricht, mit einem Absperrorgan ausgebildet ist.

## Claims

1. Bionic replacement organ having a structure comprised of three groups of textile hollow microfibers, the hollow microfibers in each group ending in their corresponding central fluid conductor, with the possibility of growing cell cultures on the outer surface of all hollow microfibers, **characterized in that** the hollow microfibers (1) in the first group are made of proton-conducting material, having perforations (2) for discharging bile into the inside of the fibers (1), and that essentially one of the surfaces of each hollow microfiber (1) of the first group is hydrophilic and lipophilic, whereas the other surface is hydrophobic and lipophobic.

2. Bionic replacement organ according to Claim 1, **characterized in that** the entire outer surfaces of the hollow microfibers are covered with a growth of human liver cells cultured *in vitro.*

3. Bionic replacement organ according to Claim 1 or 2, **characterized in that** the hollow microfibers have an inner diameter of approx. 0.1 to 50 µm.

4. Bionic replacement organ according to one of the preceding claims, **characterized in that** the hollow microfibers are arranged in a structure whose transversal section looks like a honeycomb.

5. Bionic replacement organ according to one of the preceding claims, **characterized in that** the fluid conductors are compatible with the structure of the corresponding human vessels.

6. Bionic replacement organ according to one of the preceding claims, **characterized in that** the number and size of the perforation (2) is such as to allow a bile flow of 100 to 3000 mL/day.

7. Bionic replacement organ according to one of the preceding claims, **characterized in that** the degradation of the hollow microfibers of the structure can be set to occur late or at a specific time.

8. Bionic replacement organ according to one of the preceding claims, **characterized in that** it comprises a portocaval shunt.

9. Bionic replacement organ according to one of the preceding claims, **characterized in that** the fluid conductor corresponding to the liver vein has a closing organ.

10. Bionic replacement organ according to one of the preceding claims, **characterized in that** the fluid conductor corresponding to the portal vein has a closing organ.

## Revendications

1. Organe de remplacement bionique ayant une structure comportant trois groupes de microfibres creuses, les microfibres creuses dans chaque groupe se terminant dans leur conduit de fluide central respectif, avec la possibilité de cultiver des cultures cellulaires sur la surface extérieure de toutes les microfibres creuses, **caractérisé en ce que** les microfibres creuses (1) du premier groupe sont faites d'un matériau conducteur de protons ayant des perforations (2) pour déverser la bile vers l'intérieur des fibres (1), et qu'essentiellement, une des surfaces de chaque microfibre creuse (1) du premier groupe est hydrophile et lipophile, alors que l'autre surface est hydrophobe and lipophobe.

2. Organe de remplacement bionique selon la revendication 1, **caractérisé en ce que** la totalité des surfaces extérieures des microfibres creuses est couverte de cellules hépatiques humaines cultivées *in vitro.*

3. Organe de remplacement bionique selon la revendication 1 or 2, **caractérisé en ce que** les microfibres creuses ont un diamètre intérieur d'environ 0,1 à 50 µm.

4. Organe de remplacement bionique selon l'une quelconque des revendications précédentes, **caractérisé en ce que** les microfibres creuses sont disposées pour former une structure dont la section transversale ressemble à un rayon de miel.

5. Organe de remplacement bionique selon l'une quelconque des revendications précédentes, **caractérisé en ce que** les conduits de fluide sont sont compatibles avec la structure des vaisseaux humains correspondants.

6. Organe de remplacement bionique selon l'une quelconque des revendications précédentes, **caractérisé en ce que** le nombre et la taille des perforations (2) sont tels qu'ils permettent un flux de bile de 100 à 3000 mL/jour.

7. Organe de remplacement bionique selon l'une quelconque des revendications précédentes, **caractérisé en ce que** la dégradation des microfibres creuses de la structure peut être tardive ou programmée pour un temps spécifique.

8. Organe de remplacement bionique selon l'une quelconque des revendications précédentes, **caractérisé en ce qu'**il comprend un shunt porte-cave.

9. Organe de remplacement bionique selon l'une quelconque des revendications précédentes, **caractérisé en ce que** le conduit de fluide correspondant à la veine hépatique a un organe de fermeture.

10. Organe de remplacement bionique selon l'une quelconque des revendications précédentes, **caractérisé en ce que** the conduit de fluide correspondant à la veine porte a un organe de fermeture.
